# EUROPEAN PATENT APPLICATION

(11) **EP 0 687 898 A2**
(43) Date of publication of application: **20.12.1995**
(21) Application number: 95201560.0
(22) Date of filing: 13.06.1995
(51) Int. Cl.: G01N 1/30, G01N 1/36, G01N 33/76, G01N 33/574

(54) **Differential immunocytochemical diagnosis of cancer using anti-bodies of antisera against hCG-beta**

(30) Priority: 13.06.1994 US 259044
(71) Applicant: Allegheny Health Education and Research Foundation, Pittsburgh, Pennsylvania 15222-3009 (DE); InVitro Technologies Inc., Ithaca, New York 14850 (US)
(72) Inventor: Acevedo, Hernan, Pittsburgh, Pennsylvania 15237 (US); Krichevsky, Alexander, Pittsburgh, Pennsylvania 15212 (US)
(74) Representative: Van Someren, Petronella F. H. M.

(57) **Abstract**

An immunocytochemical analytic assay for hCGβ, in a fixed sample of tissues or cells, which uniformly and reliably demonstrates the presence of hCGβ in malignant cells of all kinds. Underlying this new method is the realization that it was the omnipresent formalin fixative used in prior art immunocytochemical hCGβ assays which irretrievably destroyed the hCGβ antigens in oncologic samples and prevented their detection--as well as the realization that not one of the various known unmasking techniques had been able to revive them. The present method includes as an essential step the selection of a non-formalin fixative for use in fixing the tissue or cellular sample to be analyzed, as well as provision for staining and counterstaining to enable positive morphological classification of certain noncancerous cells known to produce hCGβ. The present protocol also reflects an underlying new appreciation that because hCGβ can be detected in malignant cells of all malignant neoplasm(s), the use of the present immunocytochemical method plus particular morphological classification of noncancer cells can positively identify malignancy in cell or tissue samples 99.9% if not 100% of the time.

## Description

### Field of the Invention

The present invention relates to the differential diagnosis of cancer using the immunocytochemistry of anti-hCGβ antisera or monoclonal antibodies, in a protocol using light microscopy examination instead of more complicated techniques.

### Background of the Invention

It is already known to use monoclonal antibodies against human chorionic gonadotropin, beta subunit (hCGβ), in a wide variety of cancer diagnostic protocols. Hormone production in cancer can result from the presence of transformed endocrine cells that continue to secrete or, as in the case of hCGβ, the hormone is considered to be an oncodevelopmental gene product and thus is generally recognized to be produced by all malignant tumors. Chapter 4 of Sell, ed., Monoclonal Antibodies in Cancer, Humana Press, Clifton, N.J., pp. 65-96 (1985), entitled "Human Chorionic Gonadotropin Detection with Monoclonal Antibodies," is directed in particular to the use of monoclonal antibodies against human chorionic gonadotropin to detect hCGβ and how the use of such antibodies differs from--and improves upon--the use of antisera against hCGβ in cancer diagnosis.

As the above-cited Chapter 4 explains, many of the potential advantages of monoclonal antibodies for clinical use are well known--availability of an unlimited supply of easily purified antibodies of uniform quality; the ability to utilize impure antigens (including cell surface proteins); and the potential for unusual specificity. The authors go on to identify monoclonal antibodies as the reagents of choice in several areas of identifying hormone cancer markers such as hCGβ.

Human chorionic gonadotropin is a glycoprotein hormone made up of an α subunit and a β subunit. The α subunit is identical with the subunit of the other glycoprotein hormones including LH, TSH and FSH, whereas the β subunit confers biological specificity with respect to receptor binding. The β subunit has a distinct carboxy terminal peptide.

Although immunoassays for hCGβ with antisera had earlier achieved good sensitivity and specificity, there still existed a need for improved assays due to the low levels of hCGβ in early pregnancy and some cancers and the occasionally high levels of LH which caused cross-reactivity. Monoclonal antibodies against hCGβ provided not only higher affinity and higher specificity but larger amounts of more easily obtained antibody for clinical diagnostic use. Monoclonal antibodies thus came into widespread use in the detection of cancer marker and other hCGβ expressions, using traditional competitive radioimmunoassays, two-site immunoradiometric assays, sandwich immunoassays (with radiolabeled and enzyme-labeled layers), flow cytometry techniques, and other methods.

One immunocytochemical method for detecting the presence of hCGβ subunits has been consistently avoided in the prior art, even though it is a much easier technique than other methods still in use. In the prior art, immunocytochemical detection of hCGβ in fixed slides was simply abandoned, because it had been uniformly determined not to work. For example, see Graeme-Cook, F., et al., "Immunocytochemical Staining for Human Chorionic Gonadotropin Subunits Does Not Predict Malignancy in Insulinomas," American Journal of Clinical Pathology, vol. 93, no. 2, pp. 273-276 (1990), whose title is as telling as its text. Graeme-Cook et al. explain how fixed and stained slides were useless to identify hCGβ marker in insulinomas, even though they admit that hCGβ had been recognized previously with other techniques in non-gonadal tumors such as those of the lung, bladder, breast and colon. The authors do not restrict their remarks to insulinomas when they point out that identification of elevated serum levels of hCGβ or the whole hCG molecule in patients with tumors suspected of malignancy are "problematic because confirmation of its [hCG's] presence within tumor cells is lacking" upon immunocytochemical analysis. Apparently, the nature of this "problematic" mystery was not resolved by these or any other authors.

Generally, immunocytochemical methods of detecting markers in biological samples are simple techniques for optical microscopy examination of conventionally prepared slides. Biopsied or otherwise collected materials are fixed and processed to allow their examination under light microscopy, and thus these methods are far easier and less expensive than analytical methods requiring complicated machines and/or computerized imaging analysis equipment. Traditionally, a tumor biopsy is divided and at least a portion of it is immediately fixed in aqueous formalin (paraformaldehyde) solution. The fixed tissue is then embedded in paraffin, cut into thin sections with a microtome, mounted, deparaffinized, washed, stained and counterstained for examination. The use of antisera and monoclonal antibodies for immunocytochemical detection can form a part of the staining protocol, so that any immune reaction visualized confirms the presence of the antigen specific for the antibody(ies) used.

As Larsson, Lars-Inge, M.D., explains in "Tissue Preparation Methods for Light Microscopic Immunohistochemistry," Applied Immunohistochemistry, vol. 1, no. 1, pp. 2-16 (1993), immunohistochemistry may in selected cases be performed on unfixed tissues. The use of unfixed tissues has been a valuable approach, he says, in identifying tightly bound or insoluble antigens in living cells, smears, imprints and fresh-frozen cryostat sections. In most cases, however, Dr. Larsson points out that specimens must be fixed before immunohistochemical staining. The purpose of such fixation is to conserve the tissue from autolysis, to inactivate contagious microorganisms, to preserve tissue and cell structures with as life-like morphology as possible, and to immobilize soluble antigens in their native cellular compartments. Unfortunately, this requires rather harsh chemical modification of the living tissue, which is not always compatible with preserving antigenic reactivity. Immunohistochemical analysis of fixed specimens is therefore (according to Dr. Larsson) a compromise situation in many cases.

The fact that fixation can destroy the very antigens that the immunohistochemical assay is intended to detect can, however, often be overcome by reviving the antigens in previously fixed cells. Dr. Larsson refers to this phenomenon as "antigen retrieval (demasking)" which can be accomplished chemically or enzymatically. Another technique for reviving antigens involves the application of microwave energy; see Gown, A., et al., "Microwave-based antigenic unmasking," Applied Immunohistochemistry, vol. 1, no. 4, pp. 256-266 (1993); see also Leong, A. et al., "An Assessment of the Efficacy of the Microwave Antigen-Retrieval Procedure on a Range of Tissue Antigens," Immunohistochemistry, vol. 1, no. 4, pp. 267-274 (1993). Notwithstanding these antigen retrieval techniques, it has heretofore been impossible to observe hCGβ binding in fixed slides **with one notable exception.** Placental cells, when fixed in formalin, embedded in paraffin, mounted, deparaffinized, stained and counterstained, demonstrate easily detectable levels of hCGβ when stained with anti-hCGβ antibody(ies) or antisera. No other cells were ever documented, in the prior art, to test "positive" for hCGβ in this way. The immunohistochemical art therefore departed wholesale from any efforts to identify hCGβ/antibody reactions in immunohistochemical reactions on microscope slides containing oncological samples. For whatever reason--in fact, for no reason known at the time--such an immunohistochemical assay for hCGβ simply could not be performed on oncological samples.

This state of affairs in the prior art was most unfortunate, due to the low cost and simplicity of immunocytochemical assays for malignant cells as compared with other more complicated and expensive methods. Accordingly, a need remained for a way--despite all traditional teaching to the contrary--to adapt the general technique of immunocytochemical analysis of a tissue sample to the hCGβ assay recognized to be so useful in other, more complicated, cancer diagnostic protocols.

### Summary of the Invention

In order to meet this need, the present invention is an immunocytochemical analytic assay for hCGβ, in a fixed sample of tissues or cells, which uniformly and reliably demonstrates the presence of hCGβ in malignant cells of all kinds. Underlying this new method is the realization that it was the omnipresent formalin fixative used in prior art immunocytochemical hCGβ assays which irretrievably destroyed the hCGβ antigens in oncologic samples and prevented their detection--as well as the realization that not one of the various known unmasking techniques had been able to revive them. (In the context of this invention, it was also identified that the reason the formalin-fixed placental slides had tested positive for hCG had to do with the inordinately high levels (100,000X an average oncologic sample) of hCG present there, which no amount of formalin could destroy.) The present method includes as an essential step the selection of a non-formalin fixative for use in fixing the tissue or cellular sample to be analyzed--preferably with the same degree of preservation as formalin was able to achieve in the prior art--as well as provision for staining and counterstaining to enable positive morphological classification of certain noncancerous cells known to produce hCGβ. The present protocol also reflects an underlying new appreciation that because hCGβ can be detected in malignant cells of all malignant neoplasm(s), the use of the present immunocytochemical method plus particular morphological classification of noncancer cells can positively identify malignancy in cell or tissue samples 99.9% if not 100% of the time.

### Brief Description of the Figures

Figure 1 shows human glioma (malignant) cells and how their red color as a result of immunocytochemical reaction with CTP103 confirms the diagnosis of malignancy.

Figures 2A and 2B show the same human glioma cells is in Figure 1, with Figure 2B showing that normal fibroblasts of the skin did not show reactivity with CTP103.

Figure 3 shows that the same cultured cells as appear in Figures 1 and 2A/2B exhibit very little reactivity to mAb A105, a mAb specific for the α subunit of hCG, not the β subunit; and

Figure 4 shows strong positive identification of malignancy of rat glioma cultured cells grown in the brain of a Wistar rat, with staining by CTP103.

### Detailed Description of the Invention

The present invention is an immunocytochemical analytic assay for hCGβ, in a fixed sample of tissues or cells, which uniformly and reliably demonstrates the presence of hCGβ in malignant cells of all kinds. Underlying this new method is the realization that it was the omnipresent formalin fixative used in prior art immunocytochemical hCGβ assays which irretrievably destroyed the hCGβ antigens in oncologic samples and prevented their detection--as well as the realization that not one of the various known unmasking techniques was able to revive them. (In the context of this invention, it was also identified that the reason the formalin-fixed placental slides had tested positive for hCGβ had to do with the inordinately high levels of hCGβ present there, which no amount of formalin could destroy.) The present method includes as an essential step the selection of a non-formalin fixative for use in fixing the tissue or cellular sample to be analyzed, as well as provision for staining and counterstaining to enable positive morphological classification of certain noncancerous cells known to produce hCGβ. The present protocol also reflects an underlying new appreciation that because hCGβ can be detected in malignant cells of all malignant neoplasm(s), the use of the present immunocytochemical method plus particular morphological classification of noncancer cells can positively identify malignancy in cell or tissue samples 99.9% if not 100% of the time.

The use of monoclonal antibodies against hCGβ for immunochemical detection of hCGβ is well known and well understood. In addition to the above-cited references, see also Wahlstrom, T., et al., "The use of monoclonal antibodies against human chorionic gonadotropin for immunoperoxidase staining of normal placenta, pituitary gland and pituitary adenomas," The Journal of Histochemistry and Cytochemistry, vol. 29, no. 2, pp. 864-865 (1981). Monoclonal antibodies against hCGβ are well known and widely available. Three exemplary monoclonal antibodies against hCGβ are CTP101, CTP102 and CTP103, hybridoma deposits for which were made on June 13, 1994 with the American Type Culture Collection (ATCC), and for which ATCC accession numbers have not yet been received but will be reported in due course. CTP101, CTP102 and CTP103 are monoclonal antibodies specific for epitopes on the carboxy terminal peptide of human chorionic gonadotropin, β subunit. Although the present method is not to be considered limited to these antibodies, they are the preferred antibodies for use in the practice of the present method.

That the well-known anti-hCGβ antisera and monoclonal antibodies should have been used, in the prior art, with tissue specimens fixed in formalin should come as no surprise to anyone skilled in traditional cytochemical and histological techniques. Formalin had been the fixative of choice for decades, for all kinds of tissue preservation but particularly for slide preparation, to the extent that its use was seldom if ever even questioned. It may be this underlying assumption which blinded those skilled in the art to its culpability in irretrievably destroying hCGβ antigen. In any event, the present innovation surprisingly identified unexpectedly good results when formalin fixatives--and comparably destructive fixatives--are avoided in the immunocytochemical identification of hCGβ in oncological samples.

The present invention does not reside simply in a method in which a non-formalin fixative is used in the preparation of a tissue sample microscope slide, instead of a formalin-based fixative. Instead, the invention requires the selection of a fixative (which will not be formalin) which does not destroy the antigenic character or hCGβ. This distinction is important. Now that the present inventors have solved the persistent prior art mystery as to why hCGβ assays could not be conducted with slides bearing fixed oncologic sections, and have identified that the present method works because it uses a fixative which does not destroy hCGβ antigenicity, those skilled in the art will be able to identify suitable fixatives in a reasonable number of tries. Even so, suitable fixatives have been identified and are discussed further supra.

Before concentrating on the particulars of the present method, it is illuminating to acknowledge the larger context of the present innovation. At the time of filing of this specification with the United States Patent and Trademark Office, the entire spectrum of the practice of medicine is undergoing fundamental change. Laboratory diagnostic reagents commonly used for decades are no longer acceptable because their use or disposal poses a biohazard to personnel and/or to the environment. Sophisticated high-tech diagnostic wizardry is coming under careful scrutiny as cost controls render it impractical and unaffordable. Even clinical diagnosis, apart from laboratory diagnosis per se, is changing hands from the specialist physician to the general or family health provider, who may or may not even be a physician at all (Certified Nurse Practitioners and Certified Nurse Midwives now perform certain diagnoses, for instance). Primary patient care, in addition to diagnosis, is changing at a basic level as health care and hospital organizational charts "flatten," as health maintenance organizations burgeon, and as the roles of health care providers become--of necessity--more flexible across the board.

The above-described trend in the medical profession is a critical backdrop to the significance of the present invention. It has been known heretofore to diagnose malignancy with complicated and expensive assays for hCGβ in or on the suspected cells. Now, however, for the first time a method has been perfected which enables diagnosis of cancer with a simple test and mere optical microscopic examination of ordinary glass microscope slides or equivalent substrates. In many cases, the tissue for examination may be simply collected from a biologic fluid or with a needle biopsy--techniques well within the skill of virtually any health care provider.

As if the above were not enough, the present health care environment is also a litigious one. Medical standards of all kinds are being reevaluated, and uniformity and standardization of diagnostic and treatment protocols has become a primary goal. Implementing this standardization in histopathology can be seen as a monumental task when viewpoints such as the following are considered. In Taylor, C., MD, "An Exaltation of Experts: Concerted Efforts in the Standardization of Immunohistochemistry," Applied Immunohistochemistry, vol. 1, no. 4, pp. 232-243 (1993), the author explains that all pathologists would probably agree that the interpretation of histologic sections is subjective, often extending through a spectrum of possibilities. The same author goes on to say that with a greater level of discrimination assayed by the pathologist, as exemplified by the histologic grading of tumors, the greater also is the degree of subjectivity--**such that the final morphologic diagnosis is often hedged with uncertainty.** Overcoming this uncertainty has proved to be an important and daunting challenge.

The potentially conflicting demands of the modern health care system, then, are drastically reduced cost **BUT** with substantially improved and even standardized reliability. In the area of cancer diagnosis, such goals might seem unachievable, except that the present technique accomplishes them all. As described more specifically below, the present method is easy and, more importantly, is 99.9% if not 100% reliable as to cancer diagnosis. It does not require the use of the increasingly disfavored formalin reagent, now acknowledged to be a mutagen and a carcinogen. The value of such a technique to the health care industry is, in light of the foregoing demands of the profession, literally difficult to overestimate.

As a practical matter, the present immunocytochemical assay includes the following steps. First, tissue to be examined is collected. The tissue may be excised by scalpel, laser or other surgical technique or may be collected by needle biopsy, "scope" biopsy or separation from body fluids. At least a small portion of the tissue collected is then fixed in a non-formalin fixative such as ULTRAFIX^{TN}, or Bouin's fixative (known in the art) or other known non-formalin fixatives (determined in a single attempt with an oncologic control positive for malignancy). Preferably, the fixative used is both nondeleterious to the hCG epitopes and capable of preserving the morphology of the cell as well as formalin traditionally did in the prior art. The sample is then generally embedded in paraffin and sectioned (usually with a microtome) onto suitable microscope slides such as the well known "Superfrost" slides which prevent tissue from sliding off. The mounted tissue sample is then deparaffinized, dried, rinsed, treated with antisera or monoclonal antibody, rinsed, stained, counterstained and examined. Because the visualization of the reactivity of hCGβ as a diagnostic assay is well known, the various known techniques for achieving a visible reaction are not enumerated here. The present invention inheres in making those known visualization reactions possible in fixed slides of cells or tissue sections, and is thus not to be limited to particular reactions or color changes.

An exemplary protocol is given in Example 1, below. The various details therein are not critical, however, except for the choice of fixative and the utilization of at least one anti-hCGβ mAb or equivalent antisera, as described above.

Preferably, when mAb(s) are used to implement the present method, more than one mAb against hCGβ is be used to identify the presence of one or more malignant cells in a given sample. Ideally, a "panel" of ten to twelve monoclonal antibodies directed to five different known binding sites of the hCGβ molecule is used. The use of a panel of mAbs is a failsafe mechanism to assure that, if there is even one malignant cell in any given sample, there is literally no possibility of its going undetected under the onslaught of an entire army of mAbs.

To examine the prepared slide for the presence of malignancy in the tissue sample, it is merely optically studied under an ordinary microscope. The morphological characteristics of the few cell types which, although not cancerous, produce hCGβ must be learned in advance, but those noncancerous cells which produce hCGβ are readily identified by their gross and unique characteristics and this skill is easily acquired and well known already in the art. When the slide is examined, therefore, any such noncancerous cells which produce hCGβ (placenta, etc.) must be identified and eliminated from consideration, and after that determination is complete the presence of any other cell positive for hCGβ indicates malignancy in the tissue under consideration. Positive and negative controls are routinely examined to confirm diagnosis.

It was previously confirmed, using flow cytometry and a battery of mAbs directed against five different sites of the hCGβ molecule, that expression of hCGβ occurred in 80 cultured human cancer cell lines of different types and origins. This work was then extended to embrace cancer cells isolated from 25 human cancerous tissues of different types obtained by elective surgery and received at in the pathology laboratory for diagnostic purposes. At the same time, analysis of cells from non-malignant neoplasms was confirmed to be negative for the presence of these hCGβ markers. These tests confirmed what was already known and what is described above, namely, that the assay for hCGβ in oncologic samples is a powerful and reliable diagnostic test for malignancies and cancers of all types.

The following example is illustrative but is not intended to be limiting.

### Example 1

In order to prepare a control, cells from culture flasks were harvested by trypsinization followed by resuspension in 15 ml serum-containing growth medium. The resuspended cells were pelleted under centrifugation for seven minutes at 200 times gravity. Cells were washed, resuspended, and repelleted. After removal of the supernatant, two-three volumes of fixative such as Ultrafix™ or Bouin's fixative were added to the pellet to resuspend it. The suspension was then stored in the refrigerator and used as needed to prepare a control slide for staining and comparative evaluation.

A tissue biopsy sample from a patient was fixed in Ultrafix™ brand fixative, embedded in paraffin and sectioned with a microtome according to techniques known in the art. Tissue sections were placed onto Superfrost Plus™ microscope slides. The slides were heated in a 60° C oven for 1 hour and then deparaffinized with two five minute washes with xylene and four 1 minute washes with absolute ethanol. The slides were air dried on a paper towel. To enhance visibility of the section, it was then circled with a "PAP-Pen™", and the slide was then rinsed in several changes of deionized water over a five minute period. The slide was then immersed in 3% aqueous hydrogen peroxide solution for five minutes, followed by an additional five minute deionized water rinse period. The slides were then placed in Tris-saline, after which excess fluid was shaken off, and normal non-immune serum was added for five minutes' incubation at room temperature.

The serum was then drained from the section, and an endogenous biotin block was effected by adding 0.1% avidin for ten minutes, washing in tris-saline three times over five minutes, adding 0.01% biotin for ten minutes, and again washing the slide in tris-saline three times over five minutes. Excess fluid was removed by shaking.

The primary antibody (or antisera) was then added to the slide, with incubation several hours or overnight at 4° C in a moist chamber. This incubation was followed by two rinses in tris-saline and one rinse in tris-Brij. Biotinylated secondary antibody was added for a thirty minute incubation period at room temperature in a moist chamber, followed by two tris-saline and one tris-Brij rinses. Avidin biotin complex was then added for a thirty minute incubation in a moist chamber, followed by three tris-saline rinses. The section was then wiped and blotted around its periphery. A very thin layer of Crystal/Mount was used to cover the section completely, and the slide was placed in a 60° C oven for one hour to set the Crystal/Mount. The slide was then cooled and "coverslipped" with Permount (Accu-Mount 60 could also have been used).

When the primary antibody or antisera used was monoclonal antibody, it was selected from among the following: CTP101, CTP102, CTP103, AS11, B108, AS12, B201, B204, B109 and B210, all of which are specific for hCGβ. For comparative testing, A105--which binds specifically with the α subunit of hCG--was also used. Secondary antibodies included biotinylated goat (F ab')₂ anti-mouse IgG (H+L) human absorbed, for use with human cells or tissues only, or biotinylated goat anti-mouse IgG (H+L) rat adsorbed, for use with rat cells or tissues only. The Tris based buffers are all known in the art.

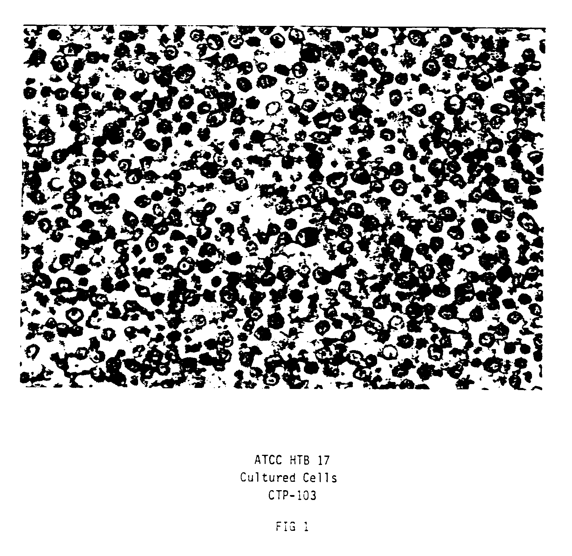

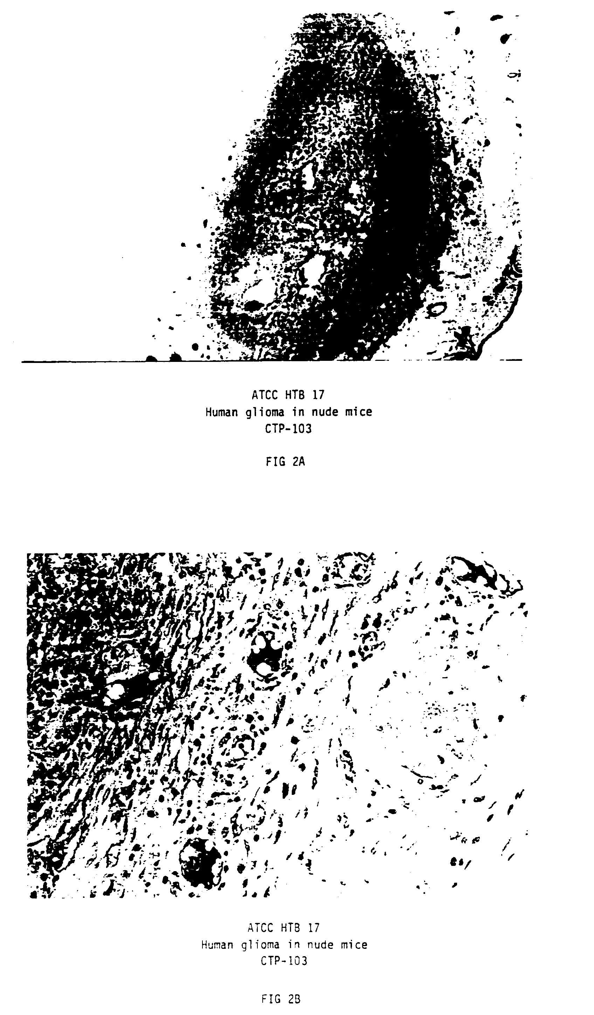

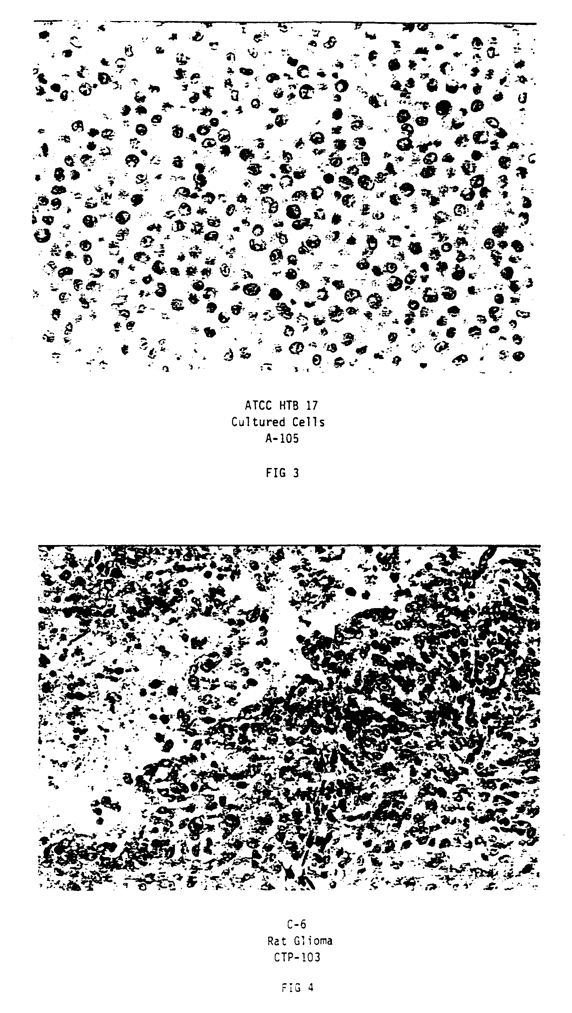

Sections prepared according to the above appear in Figures 1-4. Red/brownish staining confirms the presence of hCGβ and the presence of malignancy in the sample.

It should be noted that Bouin's fixative could have been substituted for Ultrafix, in this example. When Bouin's fixative is used, fresh fixative must be prepared on a daily basis and generally much longer washes are needed to prepare readable tissue samples, but this adaptation of the above-described method is well within the skill of the art.
Although the present invention has been described with particularity according to the foregoing description, the description is exemplary only and the invention is only to be limited insofar as is set forth in the accompanying claims.

## Claims

1. A method for diagnosing malignancy in a tissue sample, comprising the steps of:
a) fixing a tissue sample in a non-formalin fixative characterized by its ability to preserve epitopes specific to hCGβ, to create a fixed tissue sample;
b) embedding said fixed tissue sample in an immobilizing medium;
c) preparing a thin section of said fixed tissue sample embedded in immobilizing medium;
d) mounting said thin section on a substrate;
e) contacting said substrate with a series of reagents including an antibody or antisera specific for hCGβ; and
f) examining said substrate including morphological elimination of identifiable noncancerous hCGβ producing cells;
wherein the presence of visible hCGβ immune reaction in at least one noneliminated cell of said thin section confirms a diagnosis of malignancy in said thin section.

2. The method according to claim 1 wherein said immobilizing medium is paraffin and step d) includes the step of deparaffinizing said thin section.

3. The method according to claim 2 wherein said antibody or antisera specific for hCGβ is at least one monoclonal antibody selected from the group consisting of CTP101, CTP102, CTP103, AS11, B108, AS12, B201, B204, B109 and B210.

4. The method according to claim 3 wherein said series of reagents further includes at least one counterstain and several washes with ethanol and deionized water.

5. The method according to claim 4 wherein said fixative is selected from the group consisting of Ultrafix™ and Bouin's fixative.

6. The method according to claim 5 wherein said monoclonal antibody is further selected from the group consisting of CTP101, CTP102 and CTP103.

7. A test kit for the simple diagnosis of malignancy in a tissue sample, comprising a collection of at least ten monoclonal antibodies which react specifically with an epitope of hCGβ.
